# EUROPEAN PATENT SPECIFICATION

(11) **EP 3 025 666 B1**
(45) Date of publication and mention of the grant of the patent: **01.11.2017**
(21) Application number: 15196616.5
(22) Date of filing: 26.11.2015
(51) Int. Cl.: A61B 34/20, A61B 90/00

(54) **COMPUTER-ASSISTED SYSTEM FOR GUIDING A SURGICAL/DIAGNOSTIC INSTRUMENT IN THE BODY OF A PATIENT**
COMPUTERGESTÜTZTES SYSTEM ZUM FÜHREN EINES CHIRURGISCHEN/DIAGNOSTISCHEN INSTRUMENTS IM KÖRPER EINES PATIENTEN
SYSTÈME ASSISTÉ PAR ORDINATEUR PERMETTANT DE GUIDER UN INSTRUMENT DE DIAGNOSTIC/CHIRURGICAL DANS LE CORPS D'UN PATIENT

(30) Priority: 26.11.2014 IT TO20140973
(43) Date of publication of application: 01.06.2016
(73) Proprietor: MASMEC S.p.A., 70026 Modugno (IT)
(72) Inventor: LARIZZA, Piero, 70026 Modugno (IT)
(74) Representative: Bongiovanni, Simone

(56) References cited:
- WO-A1-2005/076033
- DE-A1-102008 014 270
- DE-A1-102010 013 499
- US-A1- 2006 071 135
- US-A1- 2008 077 158
- US-A1- 2011 251 625

## Description

### TECHNICAL FIELD OF THE INVENTION

The present invention relates to a computer-assisted system for guiding a surgical/diagnostic instrument in the body of a patient.

### BACKGROUND ART

As is known, many surgical and/or diagnostic operations are currently conducted with the aid of a surgical/diagnostic instrument (operating tool) able to reach, percutaneously, an area affected by a pathology (often indicated as target area). By way of example, these operations could be biopsies, thermal ablation, removal of damaged tissue, the introduction of chemical/pharmaceutical products into the human body. Introduction and subsequent reaching of the target by the surgical/diagnostic instrument can be facilitated by guide, or navigation, systems, of virtual type, based on images of the target and of the areas surrounding the target so as to plan and perform the percutaneous operation in the least invasive way.

Known navigation/guide systems for operations of the aforesaid type are based on the processing of images acquired previously through computerized tomography CT or magnetic resonance MR and require the definition of a virtual reality three-dimensional reconstruction of the target area of the human body.

A three-dimensional representation of the surgical/diagnostic instrument is superimposed on the three-dimensional reconstruction and moved on the image following the real movements of the instrument.

It is evident that in order to view the position in space of the surgical/diagnostic instrument this must be provided with suitable sensors so that the position of the surgical/diagnostic instrument is known in real time.

For example, the patent EP09425116 by the same applicant shows a computer-assisted system for guiding a surgical/diagnostic instrument in the body of a patient comprising a first marker device configured to be arranged integral with a region of the body of a patient and including first and second patient markers having a given mutual arrangement; a second marker configured to be coupled to the surgical/diagnostic instrument and including third instrument marker elements; an optical location sensor configured to locate the second and third marker elements in a first reference system; and a processing unit configured to acquire at least one tomography image of the region of the patient's body comprising the first marker elements in un second reference system different from the first, acquire the position of the second and third marker elements in the first reference system, and determine the position of said third marker elements in the second reference system based on a correlation between the first and the second reference systems.

The optical sensor generally comprises a pair of video cameras adapted to acquire, from different angles, an image of an area of vision in which the working/diagnostic instrument is located.

US2004147839 describes a method of registering an article having a surface to previously created scan data of the article, the method comprising the steps of: providing a flexible substrate having multiple tracking points attached to the substrate; replying the substrate to the article to be registered; creating a model of the surface of the article from a location of each tracking point; and registering the model with the previously created scan data.

US5999840 describes an image data registration system comprising: an image data storage unit for storing a first data set of three-dimensional image data associated with a predetermined portion of an object with reference to a first coordinate frame; a first image data acquisition and storage device for obtaining and storing a second data set of three-dimensional image data associated with a surface of said predetermined portion of said object with reference to a second coordinate frame; and a second image data acquisition and storage device for obtaining and storing a third data set of three-dimensional image data associated with a movable probe positioned in close proximity to said predetermined portion of said object with reference to a third coordinate frame; an image data processor for registering said first, second and third data sets of three-dimensional image data to generate a matched image data set of three-dimensional image data in which said first, second and third coordinate frames are relatively aligned with one another. Document US 2008/077158 A1 discloses the preamble of claim 1.

Document WO2005076033 A1 describes a device for controlling movement and method for panning a longitudinal sensor having a longitudinal axis and a measuring volume V. Said sensor comprises at least two adjacent receiving elements which are arranged on the longitudinal axis, said receiving elements being used to receive electromagnetic or acoustic signals which are outputted by transmitters which are connected to objects; a pivoting-inclining device which is connected to the sensor; a control device which enables drive devices of the pivoting-inclining device to be controlled in relation to the rotational movement about the two rotational axes.

### SUBJECT MATTER AND SUMMARY OF THE INVENTION

The object of the present invention is to improve the system described in the aforesaid patent by producing an optical sensor that can perform further functions, such as scanning functions for the detection of three-dimensional profiles.

The preceding object is achieved by the present invention as it relates to a computer-assisted system for guiding a surgical/diagnostic instrument in the body of a patient arranged on a support structure as claimed in claim 1.

### BRIEF DESCRIPTION OF THE DRAWINGS

- Fig. 1 schematically shows a computer-assisted system for guiding a surgical instrument in the body of a patient according to the present invention;
- Fig. 2 shows a first detail of the system of Fig. 1;
- Figs. 3 and 4 show a second detail of the system of Fig. 1.

### DESCRIPTION

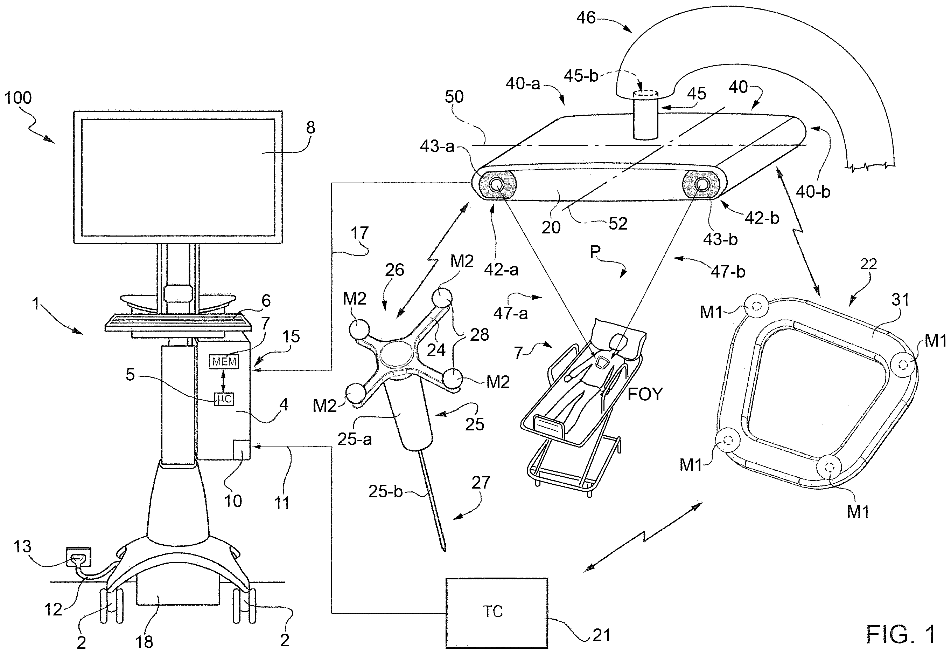

Fig. 1 shows a system according to the present invention comprising an instrumental station 1; an optical tracking sensor 20 produced according to the present invention and detailed below; in particular, an infrared sensor configured to cooperate with the instrumental station 1; a patient marker 22, provided with infrared marker elements M1 and configured to be arranged on a portion of the body of a patient P and to cooperate with the tracking sensor 20 (as better described hereunder); and an instrument marker 26, provided with a plurality of second infrared marker elements M2 (in the example spheres M2 opaque to infrared radiation that lie on the same plane and that have a predetermined arrangement relative to one another) and configured to be coupled to a surgical instrument 27 to cooperate with the infrared tracking sensor 20 (as better described below). The patient is arranged on a supporting structure 7 (typically a stretcher) that prevents shifting of the patient in space. The patient is normally anaesthetised or heavily sedated.

In particular, the surgical/diagnostic instrument 27 (in the example of Fig. 1 an endoscopic needle) comprises a grippable proximal portion 25a and an operative distal portion 25b; the instrument marker 26 is coupled to an intermediate portion, leaving the distal portion 25b free. It should be noted that, for greater clarity of representation, the relative sizes of the elements shown in the figures are not proportional to one another.

The instrumental station 1 (Fig. 1) comprises a processing unit 4, for example a processor of known type provided with a microcontroller 5 and with a memory 7, connected to each other; a data input user interface 6, for example including a keypad and mouse; a viewing interface 8, for example a high resolution LCD monitor; a network interface 10, configured to support a private and/or public network connection 11 (for example an Ethernet network).

The instrumental station 1 also comprises a power connection 12, configured to supply the instrumental station 1 with electrical power by means of a wall power socket 13; a tracking input 15, configured to support a connection 17 (either of wireless or wired type) between the instrumental station 1 and the optical tracking sensor 20; and an energy storage unit 18, for example a battery, connected to the wall socket 13 through the power connection 12 and configured to temporarily supply the instrumental station 1 in the event of an outage in the power supplied by the wall socket 13.

According to the embodiment shown, the processing unit 4 is a personal computer (PC), comprising an external protective casing housed on a shelf of the instrumental station 1 and integral with the instrumental station 1 during any movement of this latter on the wheels 2.

In order to ensure that the coordinates belonging to the surgical/diagnostic instrument 27 and acquired by the infrared/electromagnetic tracking system 20 always refer the body of the patient P, the patient marker 22 is arranged in contact with the patient so as to be integral therewith in a region of the patient (externally) close to the operating region.

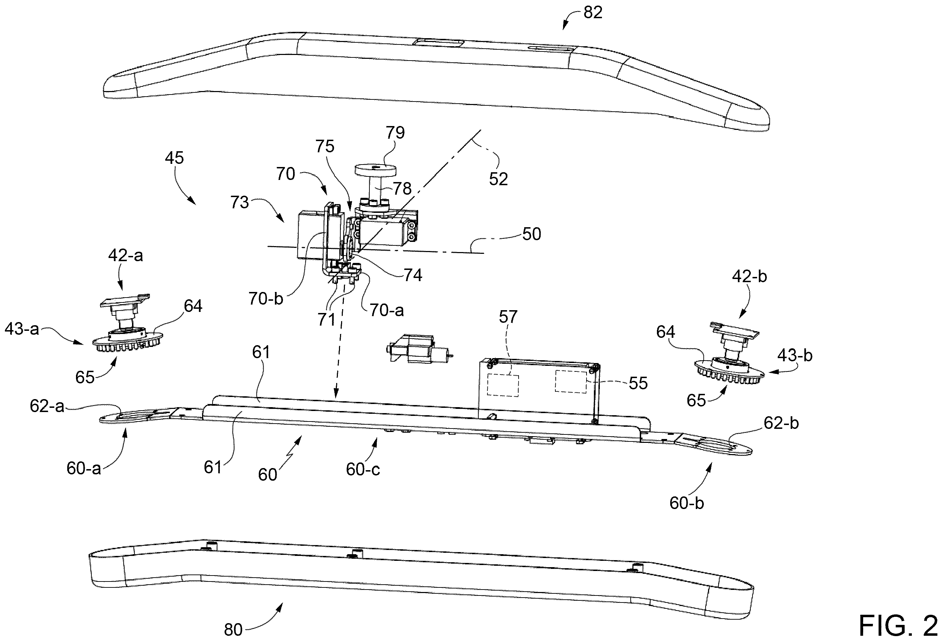

According to the embodiment shown, the patient marker 22 in the infrared version of Figs. 1 and 2, comprises a body 31, for example made of plastic material or, more in general, a material transparent to the image acquisition system used. In the example represented, the body 31 is in the shape of a trapezoidal frame with the spherical marker elements M1 arranged at the vertex of the trapezoid. Nonetheless, the shape of the marker element can differ, for example it can be Y-shaped or X-shaped, with the marker elements arranged at the ends of the arms.

The system operates as described in the patent EP09425116 by the same applicant; in particular, an initialising and calibration step is performed in which CT / MR images of an area of interest are acquired; these images are composited with one another using known algorithms in order to produce a three-dimensional image having its own reference system, the reference system of the three-dimensional image is aligned with that of the sensor 20 and, finally, an operating step is performed in which the processing unit 4 carries out a virtual navigation according to the modes described in EP09425116 using the aforesaid correctly positioned three-dimensional image as three-dimensional model.

In this way, the processing unit 4 allows the following to be viewed on the viewing unit 8:
- a three-dimensional representation of the region of the body generated based on the reconstructed and correctly positioned three-dimensional image; and
- a three-dimensional representation of the operational portion 25b of the surgical instrument 25, graphically superimposed on the three-dimensional representation of the region of the patient's body using the position of second marker elements and of the numerical model of the surgical instrument stored in the memory 7.

In this way, a system is provided that is user-friendly for the doctor oriented towards minimally invasive operations in the operating theatre.

During the phases of surgery, a three-dimensional model of the area of the patient being operated on, i.e. showing internal organs and tissues, is viewed on the viewing interface 8. As said, this three-dimensional model is generated by the processing unit 4.

In this way, it is possible to make the internal position of tissues according to the image obtained with a computerized tomography scan coherent with the current position during the operation.

The viewing interface 8 also shows a three-dimensional model of the surgical instrument used superimposed on the three-dimensional model of the area of the patient being operated on. The type of surgical instrument 25 to be viewed may be chosen by the doctor performing the operation from a plurality of possible models, previously created and stored in the memory 7 of the processing unit 4.

The doctor manipulating the surgical/diagnostic instrument 27 provided with the instrument marker 26 is guided by the images viewed on the viewing interface 8 throughout the entire operation. The trajectory of the surgical/diagnostic instrument 27 is calculated with the aid of artificial-intelligence algorithms so that the whole of the area involved is treated with the minimum number of insertions, ensuring total coverage without striking vital organs and/or obstacles. These organs are identified through the three-dimensional model shown on the viewing interface 8. The position of the surgical/diagnostic instrument 27 is entered in the three-dimensional model due to the measurement that the tracking sensor 20 performs on the spatial coordinates of the instrument marker 26.

According to the present invention, the optical tracking sensor 20 comprises (Fig. 2):
- a stereoscopic viewing system in which a first infrared ray video camera 42-a associated with a first infrared ray illuminator 43-a is carried by a first end 40-a of an elongated support element 40 and a second infrared ray video camera 42-b associated with a second infrared ray illuminator 43-b is carried by a second end 40-b of the elongated support element 40;
- a motorized pivot device 45 having a first end 45-a integral with the elongated support element 40 and a second end 45-b that can be fastened to a support structure 46 (partially shown in Fig. 1) adapted to maintain the sensor 20 in a high position above the stretcher 7 on which the patient P is lying.

The video cameras 42-a and 42-b have respective axes 47-a and 47-b tilted towards each other and converging in an area of view FOV in which the patient P is located.

The motorized pivot device 45 has at least two degrees of freedom and produces the rotation of the support element 40 relative to the support structure 46 about a first horizontal axis 50 (tilt) and according to a second axis 52 (pan) perpendicular to the first. The instantaneous value of the angle **alpha** (relative to a vertical reference) about the tilt axis 50 and the instantaneous value of the angle **beta** (relative to a vertical reference) about the pan axis is detected by a solid state inertial sensor (inclinometer) 55 (of known type - Fig. 2) arranged on the support element 40. The signal produced by the inertial sensor 55 is sent to a microprocessor control unit 57 carried by the support element 40 and adapted to drive the actuators (shown below) that produce the rotations about the tilt axis 50 and the pan axis 52.

Control of the actuators that produce the movement of the pivot device about the tilt axis 50 and about the pan axis 52 enables the area of vision FOV to be moved in space; in particular - as will be explained in more detail below - the area of vision FOV is moved so as to maintain the image of the instrument marker 26 detected by the video camera 42-a/42-b substantially at the centre of the image detected also in the event of shifting of the instrument 27 in order to perform a tracking function of the instrument marker 26 (and therefore of the surgical/diagnostic instrument 27) by the optical tracking sensor 20.

The implementation of movements about the tilt 50 and pan 52 axes enables the sensor 20 to track the operating tool so that it is always in the FOV, i.e. in the area in which the errors are lowest.

In greater detail (Fig. 2), the support element 40 comprises an elongated metal wall 60 having a pair of integral lips 61 that extend along the longer sides of a flat rectangular central portion 60-c of the elongated wall 60. The elongated wall 60 has end portions 60-a, 60-b of the elongated wall 60 of flat type integral with the central portion 60-c and arranged folded by a few degrees relative to the central portion 60-c. Each end portion 60-a, 60-b is provided with a circular through hole 62-a, 62-b in which there is housed a respective assembly comprising the first/second video camera 42-a, 42-b and the first/second illuminator 43-a,43-b. For example, the unit can comprise a flat wall 64 in the shape of a circular crown supporting, on a first face thereof, the LEDs 65 of the illuminator and provided with a video camera mounted coaxially to the circular crown shaped flat structure 64 (the axis of the video camera is coaxial to the circular crown).

The first end of the pivot element 45 is stably connected with the central portion 60-c.

The pivot device 45 comprises a first L-shaped bracket 70 provided with a first flat portion with a shorter side 70-a fastened by means of screws 71 on the central portion 60-b and a second flat portion with a longer side 70-b that carries a first actuator 73 of electrical type provided with a first rotating output member 74 that rotates about the first tilt axis 50.

An end portion of the first rotating output member 74 is fixed to a first flat portion of a second L-shaped bracket 75 parallel to the second flat portion 70-b. The second L-shaped bracket 75 comprises a second flat portion perpendicular to the first and fixed to an end portion of a second rotating output member (not visible in the figures) of a second electrical actuator 77. The second rotating output member extends from a casing of the second actuator 77 and rotates about the pan axis 52. The casing of the second actuator 77 is integral with a tubular sleeve 78, the circular end flange 79 of which produces the second end of the pivot 45. The pivot device 45 produced according to the present invention comprises a limited number of parts and for this reason is simple to produce, is rustic and sturdy and has limited costs. Moreover, the sizes of the pivot device 45 are limited.

The sensor 20 comprises an external protective casing that houses the metal wall 60 and the elements carried thereby. The protective casing comprises a lower shell 80 adapted to be fixed to a lower face of the wall 60 and provided with end holes (not shown) for the video cameras 42-a, 42-b and an upper half-shell 82 adapted to be fixed to an upper face of the wall 60 and provided with a central hole 84 engaged, in use, by the sleeve 78.

Preferably, the image processing operations for driving the actuators 73 and 77 are as follows:
a) two-dimensional coordinates (xa1,ya1 - xa2,ya2 - xa3,ya3) of the centre of the markers M2 (spheres) are determined on the images detected by the video cameras 42-a, 42-b as the single sphere that produces the marker M is viewed as a point in space;
b) the two images are composited to determine the three-dimensional coordinates (in the example a triplet xa1,ya1,za1 - xa2,ya2,za2 - xa3,ya3,za3 although this number can be different, for example four xa1,ya1,za1 - xa2,ya2,za2 - xa3,ya3,za3 - xa4,ya4,za4) of the centre of the markers M2 in space;
c) on the basis of the three-dimensional coordinates (xa1, ya1, za1 - xa2,ya2,za2 - xa3,ya3,za3) defined, the centroid of the markers and the three angles that together with the three coordinates identify the 6 degrees of freedom (DOF) that characterise the position of a rigid body (instrument marker 26) in space are calculated;
d) the operations a), b) and c) are repeated iteratively at successive instants so as to define the instantaneous value of a vector V that provides the position of the centroid and the direction and sense of its shift in space;
e) the position of the vector is compared with a three-dimensional threshold that defines a cuboid within which the vector must be contained to enable a good view of the surgical/diagnostic instrument in the FOV; if the three-dimensional threshold is not respected, the actuators are controlled to produce a shift of the centroid along the same direction and with a sense opposite to the one defined by the vector. The extent of the shift is such as to return the centroid within the three-dimensional threshold and enables good view of the surgical/diagnostic instrument in the FOV.

According to a further feature , the tracking sensor is also provided with:
- a first generator 90 adapted to emit a laser beam LS along a first direction transverse to the elongated support element 40 and that intersects the area of view FOV. The laser beam LS has a wavelength visible to the human eye and is provided to produce a laser spot (for example in the shape of a cross) visible on the body of the patient P that can be used in a procedure for adjustment of the sensor 20;
- a second generator 92 adapted to emit an infrared beam IR along a second direction transverse to the elongated support element 40 and that intersects the area of view FOV affecting the patient P. There is also provided a deflection device that produces the movement of the infrared beam IR relative to the support structure 7 (the stretcher 7 in the example) so as to produce an IR spot (visible by the video cameras) that hits an area of the patient and moves in space performing the scan of this Preferably, the deflection device can also produce the movement of the laser beam to move the laser spot on the patient's body.

The video cameras 42-a, 42-b are adapted to detect the image of the IR spot to detect (by means of algorithms of known type and therefore not further detailed) the distance between sensor 20 and spot and the position in space (x,y,z) of this spot. The control unit 57 is adapted to correlate the distance values measured for successive adjacent positions of the IR spot and repeat the scans for sections contiguous with one another (typically parallel) in order to obtain a three-dimensional profile of the area of interest.

According to a first variant of embodiment, the infrared ray generator is integral with the support element 40 and emits an IR laser beam having a fixed predetermined tilt relative to the support element 40. In this case, the deflection device is produced by the pivot device 45 that performs the rotation of the support element 40 about the tilt 50 and pan 52 axes to adjust the position in space of the IR beam and move the IR spot to the area of the patient in order to perform a scan. The video cameras 42-a, 42-b detect the image of the IR spot and determine the distance between the sensor 20 and the position in space (x,y,z) of this spot. On the basis of these data, a three-dimensional profile of the area involved is obtained (by means of known algorithms) and stored.

In this way, the pan and tilt movements are exploited to obtain the profile of the area of interest of the patient. These profiles can be compared, by means of known comparison algorithms, with stored profiles obtained by previously detected CT/MR images. This avoids the use of manual trackers or trackers that require to be aligned with the coordinate system of the sensor 20. In fact, the reference between tracking sensor and viewing sensor is direct, as both functions are performed by the same device (the sensor 20).

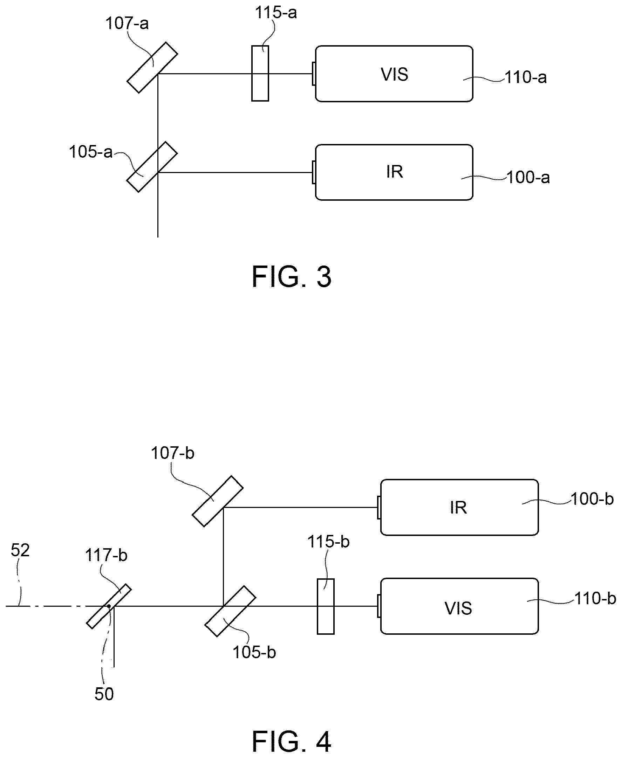

Fig. 3 shows in detail the optical system of this first embodiment of the invention, which comprises a plurality of components carried by the support structure 46, in particular:
- a source 100-a configured to emit a beam of infrared rays that is fed to a first input of a beam splitter 105-a;
- a laser source 110-b that emits a beam of visible light that is fed to a second input of the beam splitter 105-a after having passed through a pattern generator 115-a (for example the pattern produced can be a cross as indicated above) and has been reflected by 90° by a mirror 107-a.

The beam splitter 105-a is configured to output the beam of infrared rays and the laser beam having directions of propagation parallel to each other.

These beams move in space thanks to the movement of the pivot device 45.

According to an alternative embodiment of the invention shown in Fig. 4, the infrared ray generator 90 is integral with the support element 40 and emits an IR laser beam having an adjustable tilt relative to the support element 40 by means of an optical deflection system. In this case, the pivot device 45 is not used to perform the scan as the optical deflection system adjusts the position in space of the IR beam and moves the IR spot to the area of the patient performing a scan. The video cameras 42-a, 42-b detect the image of the IR spot and determine the distance between the sensor 20 and the position in space (x,y,z) of this spot. On the basis of these data, a three-dimensional profile of the area involved is obtained and stored.

With particular reference to Fig. 4, the infrared ray generator 90 produced according to this alternative embodiment comprises:
- a source 100-b configured to emit a beam of infrared rays that is fed to a first input of a beam splitter 105-b after being reflected by a mirror 107-b for infrared rays arranged tilted by 45° relative to the direction of propagation of the ray;
- a laser source 110-b that emits a beam of visible light that is fed to a second input of the beam splitter 105-b after having passed through a pattern generator 115-b (for example the pattern produced can be a cross, as indicated above); the beam splitter 105-b is configured to output the beam of infrared rays and the laser beam having directions of propagation parallel to each other;
- an oscillating mirror 117-b on which the infrared beam and the laser beam coming from the output of the beam splitter are incident. The oscillating mirror 117-b is configured to swivel about the horizontal tilt axis 50 and about the pan axis 52.
- Typically the oscillating mirror 117-b is produced using MEMS technology.

## Claims

1. Computer-assisted system for guiding a surgical/diagnostic instrument (27) in the body of a patient arranged on a supporting structure (7) comprising:
- a first patient marker device (22) configured to be arranged in contact with the patient so as to be integral with a body region of a patient (P) to be treated by means of said surgical/diagnostic instrument (27) and including first marker elements (M1);
- a second instrument marker device (26) configured to be coupled to said surgical/diagnostic instrument (27) and including second marker elements (M2);
- an optical location sensor (20) configured to locate said first and second marker elements (M1,M2); and
- a processing unit (4) connected to the optical location sensor (20) and adapted to perform a virtual navigation based on an imported/reconstructed three-dimensional image and on the image detected by the optical location sensor (20) for viewing on a viewing unit (8);
- a three-dimensional representation of the body region generated based on said three-dimensional image repositioned relative to the optical location sensor (20) reference system; and
- a three-dimensional representation of at least one operating portion (25b) of the surgical/diagnostic instrument (27), graphically superimposed on the three-dimensional representation of the body region of the patient using the position of the second marker elements (M2) and of a model of the surgical instrument, the optical location sensor (20) comprising:
- a stereoscopic viewing system in which a first infrared ray video camera (42-a) associated with a first infrared ray illuminator (43-a) is carried by a first end (40-a) of an elongated support element (40) and a second infrared ray video camera (42-b) associated with a second infrared ray illuminator (43-b) is carried by a second end (40-b) of the elongated support element (40),
- the video cameras (42-a and 42-b) have respective axes (47-a and 47-b) tilted towards each other and converging in an area of view (FOV) of the optimal location sensor (20) ;
- **characterised in that** the optical location sensor (20) comprises a motorized pivot device (45) having a first end (45-a) integral with the elongated support element (40) and a second end (45-b) that can be fastened to a support structure (46);
said motorized pivot device (45) having **at least two degrees of freedom and** being configured to produce the rotation of the elongated support element (40) relative to the support structure (46) about a first horizontal tilt axis (50) and according to a second horizontal pan axis (52) perpendicular to the first horizontal tilt axis (50);
and **in that** the optical location sensor (20) comprises a microprocessor control unit (57) configured to drive the actuators that produce the rotations about the first horizontal tilt axis (50) and the second horizontal pan axis (52) to move the area of view in space;
the microprocessor control unit (57) being configured to move the area of view and maintain the image of the marker instrument (26) detected by the video camera (42-a/42-b) substantially at the centre of the same image also in the case of shifting of the instrument in order to perform a tracking function of the instrument marker by the optical location sensor (20).

2. System according to claim 1, wherein there is provided an inertial sensor (55), in particular an inclinometer, carried by the elongated support element (40) and adapted to detect the instantaneous value of an angle **alpha** about the first horizontal tilt axis (50) and the instantaneous value of an angle **beta** about the second horizontal pan axis (52) relative to a reference; the signal produced by the inertial sensor (55) is sent to said microprocessor control unit (57).

3. System according to claim 1 or 2, wherein the microprocessor control unit (57) is carried by the elongated support element (40).

4. System according to any one of the preceding claims, wherein the elongated support element (40) comprises an elongated wall (60) comprising a flat rectangular central portion (60-c) and flat end portions (60-a, 60-b) arranged tilted relative to the central portion (60-c); each end portion (60-a, 60-b) is provided with a through hole (62-a, 62-b) in which there is housed a respective assembly comprising the first/second video camera (42-a, 42-b) and the first/second illuminator (43-a,43-b); the first end of the motorized pivot device (45) is stably connected with the central portion (60-c).

5. System according to claim 4, wherein each assembly comprises a flat support wall (64) in the shape of a circular crown supporting, on a first face thereof, the LEDs (65) of the illuminator and provided with a video camera mounted coaxially to the circular crown shaped flat support wall (64).

6. System according to claim 4 or 5, wherein the motorized pivot device (45) comprises a first L-shaped bracket (70) provided with a first flat portion with a shorter side (70-a) fastened on the central portion (60-b) and a second flat portion with a longer side (70-b) that carries a first actuator (73) of electrical type provided with a first rotating output member (74) that rotates about the first horizontal tilt axis (50); an end portion of the first rotating output member (74) is fixed to a first flat portion of a second bracket (75) and that comprises a second flat portion perpendicular to the first and fixed to an end portion of a second rotating output member of a second electrical actuator (77); the second rotating output member extends from a casing of the second actuator (77) and rotates about the second horizontal pan axis (52); the casing of the second actuator (77) is integral with an element that produces the second end of the motorized pivot device (45).

7. System according to any one of the preceding claims, wherein said optical location sensor (20) is provided with a first generator adapted to emit a laser beam LS along a first direction that is transverse to the elongated support element (40) and intersects the area of view (FOV); the laser beam LS has a wavelength visible to the human eye and is provided to produce a laser spot visible on the body of the patient P that can be used in a procedure for adjustment of the optical location sensor (20).

8. System according to claim 7, wherein said optical location sensor (20) is provided with a second generator adapted to emit an infrared beam along a second direction that is transverse to the elongated support element (40) and intersects the area of view (FOV) affecting the patient (P);
the system also comprises a deflection device that produces the movement of the infrared beam relative to the support structure (7) so as to produce an infrared spot that hits an area of the patient and moves in space performing the scan of this area; said video cameras (42-a, 42-b) are adapted to detect the position in space (x,y,z) of this spot and the control unit detects the distance between optical location sensor (20) and position in space (x,y,z) of the spot; the control unit (57) is adapted to correlate the distance values measured for successive adjacent positions of the spot and repeat the scans for sections contiguous with one another in order to obtain a three-dimensional profile of the area of interest.

9. System according to claim 8, wherein the second infrared ray generator belongs to an optical system integral with the elongated support element (40) and emits an infrared beam having a predetermined tilt relative to the elongated support element (40); the deflection device is produced by the motorized pivot device (45) that performs the rotation of the elongated support element (40) about the first horizontal tilt (50) and the second horizontal pan (52) axes to adjust the position in space of the infrared beam and move the infrared spot to the area of the patient in order to perform a scan.

10. System according to claim 9, wherein the optical system comprises:
- a source (100-a) configured to emit an infrared beam that is fed to a first input of a beam splitter (105-a);
- a laser source (110-b) that emits a visible light beam that is fed to a second input of the beam splitter (105-a) after having passed through a pattern generator (115-a); the beam splitter (105-a) is configured to output the beam of infrared rays and the laser beam having directions of propagation parallel to each other.

11. System according to claim 8, wherein the infrared ray generator is integral with the elongated support element (40) and emits an infrared beam having an adjustable tilt relative to the elongated support element (40) by means of the deflection produced by an optical system carried by the elongated support element (40) and configured to adjust the position in space of the infrared beam move the infrared spot on the area of the patient performing a scan.

12. System according to claim 11, wherein the second generator comprises:
- a source (100-b) configured to emit a beam of infrared rays that is fed to a first input of a beam splitter (105-b);
- a laser source (110-b) that emits a beam of visible light that is fed to a second input of the beam splitter (105-b); the beam splitter (105-b) is configured to output the beam of infrared rays and the laser beam having directions of propagation parallel to each other;
- an oscillating mirror (117-b) on which the infrared beam and the laser beam coming from the output of the beam splitter are incident; the oscillating mirror is configured to swivel about the first horizontal tilt axis (50) and about second the horizontal pan axis (52).

13. System according to claim 12, wherein a pattern generator (115-b) is interposed between the laser source (110-b) and the second input of the beam splitter (105-b).

## Patentansprüche

1. Computergestütztes System zum Führen eines chirurgischen/diagnostischen Instruments (27) im Körper eines auf einer Tragkonstruktion (7) angeordneten Patienten, das aufweist:
eine erste Patient-Markervorrichtung (22), die so konfiguriert ist, dass sie in Kontakt mit dem Patient angeordnet ist, um in eine Körperregion eines mit Hilfe des chirurgischen/diagnostischen Instruments (27) zu behandelnden Patienten (P) integriert zu sein, und erste Markerelemente (M1) aufweist;
eine zweite Instrument-Markervorrichtung (26), die so konfiguriert ist, dass sie mit dem chirurgischen/diagnostischen Instrument (27) gekoppelt ist, und zweite Markerelemente (M2) aufweist;
einen optischen Lagesensor (20), der so konfiguriert ist, dass er die ersten und zweiten Markerelemente (M1, M2) ortet; und
eine Verarbeitungseinheit (4), die mit dem optischen Lagesensor (20) verbunden und geeignet ist, eine virtuelle Navigation auf der Grundlage eines importierten/rekonstruierten dreidimensionalen Bilds und auf dem durch den optischen Lagesensor (20) detektierten Bild zur Betrachtung auf einer Betrachtungseinheit (8) durchzuführen;
wobei eine dreidimensionale Darstellung der Körperregion, die auf der Grundlage des dreidimensionalen Bilds erzeugt ist, relativ zum Referenzsystem des optischen Lagesensors (20) repositioniert ist; und
eine dreidimensionale Darstellung mindestens eines Arbeitsabschnitts (25b) des chirurgischen/diagnostischen Instruments (27) der dreidimensionalen Darstellung der Körperregion des Patienten mit Hilfe der Position der zweiten Markerelemente (M2) und eines Modells des chirurgischen Instruments überlagert ist,
wobei der optische Lagesensor (20) aufweist:
ein stereoskopisches Betrachtungssystem, in dem eine erste Infrarotvideokamera (42-a) in Zuordnung zu einem ersten Infrarotilluminator (43-a) durch ein erstes Ende (40-a) eines länglichen Tragelements (40) mitgeführt wird und eine zweite Infrarotvideokamera (42-b) in Zuordnung zu einem zweiten Infrarotilluminator (43-b) durch ein zweites Ende (40-b) des länglichen Tragelements (40) mitgeführt wird, die Videokameras (42-a und 42-b) jeweilige Achsen (47-a und 47-b) haben, die zueinander gekippt sind und in einem Sichtbereich (FOV) des optischen Lagesensors (20) konvergieren;
**dadurch gekennzeichnet, dass** der optische Lagesensor (20) eine motorisierte Drehgelenkvorrichtung (45) aufweist, die ein erstes Ende (45-a), das in das längliche Tragelement (40) integriert ist, und ein zweites Ende (45-b) hat, das an einer Tragkonstruktion (46) befestigt sein kann; wobei die motorisierte Drehgelenkvorrichtung (45) mindestens zwei Freiheitsgrade hat und so konfiguriert ist, dass sie die Drehung des länglichen Tragelements (40) relativ zur Tragkonstruktion (46) um eine erste horizontale Kippachse (50) und gemäß einer zweiten horizontalen Schwenkachse (52) erzeugt, die senkrecht zur ersten horizontalen Kippachse (50) ist;
und dadurch, dass der optische Lagesensor (20) eine Mikroprozessor-Steuereinheit (57) aufweist, die so konfiguriert ist, dass sie die Aktoren antreibt, die die Drehungen um die erste horizontale Kippachse (50) und die zweite horizontale Schwenkachse (52) erzeugen, um den Sichtbereich räumlich zu bewegen; wobei die Mikroprozessor-Steuereinheit (57) so konfiguriert ist, dass sie den Sichtbereich bewegt und das durch die Videokamera (42-a/42-b) detektierte Bild des Markerinstruments (26) im Wesentlichen in der Mitte dieses Bilds auch bei Verschiebung des Instruments hält, um eine Verfolgungsfunktion des Instrumentenmarkers durch den optischen Lagesensor (20) durchzuführen.

2. System nach Anspruch 1, wobei ein Trägheitssensor (55), insbesondere ein Neigungsmesser, vorgesehen ist, der durch das längliche Tragelement (40) mitgeführt wird und geeignet ist, den Momentanwert eines Winkels Alpha um die erste horizontale Kippachse (50) und den Momentanwert eines Winkels Beta um die zweite horizontale Schwenkachse (52) relativ zu einer Referenz zu detektieren; wobei das durch den Trägheitssensor (55) erzeugte Signal zur Mikroprozessor-Steuereinheit (57) gesendet wird.

3. System nach Anspruch 1 oder 2, wobei die Mikroprozessor-Steuereinheit (57) durch das längliche Tragelement (40) mitgeführt wird.

4. System nach einem der vorstehenden Ansprüche, wobei das längliche Tragelement (40) eine längliche Wand (60) mit einem ebenen rechtwinkligen Mittelabschnitt (60-c) und ebenen Endabschnitten (60-a, 60-b) aufweist, die relativ zum Mittelabschnitt (60-c) gekippt angeordnet sind; wobei jeder Endabschnitt (60-a, 60-b) mit einem Durchgangsloch (62-a, 62-b) versehen ist, in dem eine jeweilige Anordnung mit der ersten/zweiten Videokamera (42-a, 42-b) und dem ersten/zweiten Illuminator (43-a, 43-b) untergebracht ist; und das erste Ende der motorisierten Drehgelenkvorrichtung (45) mit dem Mittelabschnitt (60-c) stabil verbunden ist.

5. System nach Anspruch 4, wobei jede Anordnung eine ebene Tragwand (64) in Form einer Kreiskrone aufweist, die auf einer ersten Fläche davon die LEDs (65) des Illuminators trägt und mit einer Videokamera versehen ist, die koaxial zur kreiskronenförmigen ebenen Tragwand (64) angebaut ist.

6. System nach Anspruch 4 oder 5, wobei die motorisierte Drehgelenkvorrichtung (45) eine erste L-förmige Halterung (70) aufweist, die mit einem ersten ebenen Abschnitt mit einer kürzeren Seite (70-a), die am Mittelabschnitt (60-b) befestigt ist, und einem zweiten ebenen Abschnitt mit einer längeren Seite (70-b) versehen ist, die einen ersten Aktor (73) vom elektrischen Typ trägt, der mit einem ersten Drehausgabeteil (74) versehen ist, das um die erste horizontale Kippachse (50) dreht; wobei ein Endabschnitt des ersten Drehausgabeteils (74) an einem ersten ebenen Abschnitt einer zweiten Halterung (75) fixiert ist, die einen zweiten ebenen Abschnitt senkrecht zum ersten aufweist und an einem Endabschnitt eines zweiten Drehausgabeteils eines zweiten elektrischen Aktors (77) fixiert ist; sich das zweite Drehausgabeteil von einem Gehäuse des zweiten Aktors (77) erstreckt und um die zweite horizontale Schwenkachse (52) dreht; und das Gehäuse des zweiten Aktors (77) in ein Element integriert ist, das das zweite Ende der motorisierten Drehgelenkvorrichtung (45) bildet.

7. System nach einem der vorstehenden Ansprüche, wobei der optische Lagesensor (20) mit einem ersten Generator versehen ist, der geeignet ist, einen Laserstrahl LS in einer ersten Richtung abzustrahlen, die quer zum länglichen Tragelement (40) ist und den Sichtbereich (FOV) schneidet; wobei der Laserstrahl LS eine für das menschliche Auge sichtbare Wellenlänge hat und so bereitgestellt wird, dass er einen auf dem Körper des Patienten P sichtbaren Laserpunkt erzeugt, der in einem Verfahrensablauf zur Einstellung des optischen Lagesensors (20) verwendet werden kann.

8. System nach Anspruch 7, wobei der optische Lagesensor (20) mit einem zweiten Generator versehen ist, der geeignet ist, einen Infrarotstrahl in einer zweiten Richtung abzustrahlen, die quer zum länglichen Tragelement (40) ist und den Sichtbereich (FOV) schneidet, der sich auf den Patient (P) auswirkt; wobei das System ferner eine Ablenkvorrichtung aufweist, die die Bewegung des Infrarotstrahls relativ zur Tragkonstruktion (7) erzeugt, um einen Infrarotpunkt zu erzeugen, der auf einen Bereich des Patienten trifft und sich bei Durchführung des Scans dieses Bereichs räumlich bewegt; die Videokameras (42-a, 42-b) geeignet sind, die räumliche Position (x, y, z) dieses Punkts zu detektieren, und die Steuereinheit den Abstand zwischen dem optischen Lagesensor (20) und der räumlichen Position (x, y, z) des Punkts detektiert; die Steuereinheit (57) geeignet ist, die für aufeinanderfolgende benachbarte Positionen des Punkts gemessenen Abstandswerte zu korrelieren und die Scans für aneinander angrenzende Teilstücke zu wiederholen, um ein dreidimensionales Profil des interessierenden Bereichs zu erhalten.

9. System nach Anspruch 8, wobei der zweite Infrarotgenerator zu einem optischen System gehört, das in das längliche Tragelement (40) integriert ist und einen Infrarotstrahl mit einer vorbestimmten Neigung relativ zum länglichen Tragelement (40) abstrahlt; wobei die Ablenkvorrichtung durch die motorisierte Drehgelenkvorrichtung (45) gebildet ist, die die Drehung des länglichen Tragelements (40) um die erste horizontale Kipp- (50) und die zweite horizontale Schwenkachse (52) durchführt, um die räumliche Position des Infrarotstrahls einzustellen und den Infrarotpunkt zum Bereich des Patienten zu bewegen, um einen Scan durchzuführen.

10. System nach Anspruch 9, wobei das optische System aufweist:
eine Quelle (100-a), die so konfiguriert ist, dass sie einen Infrarotstrahl abstrahlt, der einem ersten Eingang eines Strahlteilers (105-a) zugeführt wird;
eine Laserquelle (110-b), die einen sichtbaren Lichtstrahl abstrahlt, der einem zweiten Eingang des Strahlteilers (105-a) nach Durchlaufen eines Mustergenerators (115-a) zugeführt wird; wobei der Strahlteiler (105-a) so konfiguriert ist, dass er den Infrarotstrahl und den Laserstrahl mit zueinander parallelen Ausbreitungsrichtungen ausgibt.

11. System nach Anspruch 8, wobei der Infrarotgenerator in das längliche Tragelement (40) integriert ist und einen Infrarotstrahl mit einer einstellbaren Neigung relativ zum länglichen Tragelement (40) mit Hilfe der Ablenkung abstrahlt, die durch ein optisches System erzeugt wird, das durch das längliche Tragelement (40) mitgeführt wird und so konfiguriert ist, dass es die räumliche Position des Infrarotstrahls einstellt, um den Infrarotpunkt auf dem Bereich des Patienten bei Durchführung eines Scans zu bewegen.

12. System nach Anspruch 11, wobei der zweite Generator aufweist:
eine Quelle (100-b), die so konfiguriert ist, dass sie Infrarotstrahlen abstrahlt, die einem ersten Eingang eines Strahlteilers (105-b) zugeführt werden;
eine Laserquelle (110-b), die einen sichtbaren Lichtstrahl abstrahlt, der einem zweiten Eingang des Strahlteilers (105-b) zugeführt wird; wobei der Strahlteiler (105-b) so konfiguriert ist, dass er die Infrarotstrahlen und den Laserstrahl mit zueinander parallelen Ausbreitungsrichtungen ausgibt;
einen Schwingspiegel (117-b), auf den der Infrarotstrahl und der Laserstrahl auftreffen, die aus dem Ausgang des Strahlteilers stammen; wobei der Schwingspiegel so konfiguriert ist, dass er um die erste horizontale Kippachse (50) und um die zweite horizontale Schwenkachse (52) schwenkt.

13. System nach Anspruch 12, wobei ein Mustergenerator (115-b) zwischen der Laserquelle (110-b) und dem zweiten Eingang des Strahlteilers (105-b) eingefügt ist.

## Revendications

1. Système assisté par ordinateur pour guider un instrument chirurgical / de diagnostic (27) dans le corps d'un patient disposé sur une structure de support (7) comprenant:
- un premier dispositif marqueur de patient (22) configuré pour être agencé en contact avec le patient de manière à être entièrement avec une région du corps d'un patient (P) à traiter au moyen dudit instrument chirurgical / de diagnostic (27) et comprenant des premiers éléments marqueurs (M1);
- un second dispositif marqueur d'instrument (26) configuré pour être couplé audit instrument chirurgical / de diagnostic (27) et comprenant des seconds éléments marqueurs (M2);
- un capteur de localisation optique (20) configuré pour localiser lesdits premier et second éléments marqueurs (M1, M2); et
- une unité de traitement (4) connectée au capteur optique de localisation (20) et adaptée pour effectuer une navigation virtuelle basée sur une image tridimensionnelle importée / reconstruite et sur l'image détectée par le capteur optique de localisation (20) pour visualiser sur une unité de visualisation (8);
- une représentation tridimensionnelle de la région du corps générée sur la base de ladite image tridimensionnelle repositionnée par rapport au système de référence du capteur optique de localisation (20); et
- une représentation tridimensionnelle d'au moins une partie opérationnelle (25b) de l'instrument chirurgical / diagnostic (27), superposée graphiquement sur la représentation tridimensionnelle de la région du corps du patient en utilisant la position des seconds éléments marqueurs (M2) et d'un modèle de l'instrument chirurgical, le capteur optique de localisation (20) comprenant :
- un système de visualisation stéréoscopique dans lequel une première caméra vidéo à rayons infrarouges (42-a) associée à un premier illuminant à rayons infrarouges (43-a) est porté par une première extrémité (40-a) d'un élément de support allongé (40) et une seconde caméra vidéo à rayons infrarouges (42-b) associée à un second illuminant à rayons infrarouges (43-b) est portée par une seconde extrémité (40- b) de l'élément de support allongé (40),
- les caméras vidéo (42-a et 42-b) ayant des axes respectifs (47-a et 47-b) inclinés l'un vers l'autre et convergeant dans une zone de visualisation (FOV) du capteur optique de localisation (20);
- **caractérisé en ce que** le capteur optique de localisation (20) comprend un dispositif de pivotement motorisé (45) ayant une première extrémité (45-a) solidaire de l'élément de support allongé (40) et une seconde extrémité (45-b) qui peut être fixée à une structure de support (46); ledit dispositif de pivotement motorisé (45) ayant au moins deux degrés de liberté et étant configuré pour produire la rotation de l'élément de support allongé (40) par rapport à la structure de support (46) autour d'un premier axe horizontal d'inclinaison (50) et selon un second axe horizontal panoramique (52) perpendiculaire au premier axe horizontal d'inclinaison (50);
et **en ce que** le capteur optique de localisation (20) comporte une unité de commande à microprocesseur (57) configurée pour entraîner les actionneurs qui produisent des rotations autour du premier axe horizontal d'inclinaison (50) et le second axe horizontal panoramique (52) pour déplacer la zone de visualisation dans l'espace; l'unité de commande à microprocesseur (57) étant configurée pour déplacer la zone de visualisation et maintenir l'image du marqueur d'instrument (26) détectée par la caméra vidéo (42-a / 42-b) sensiblement au centre de la même image également dans le cas du décalage de l'instrument afin d'effectuer une fonction de suivi du marqueur d'instrument par le capteur optique de localisation (20).

2. Système selon la revendication 1, dans lequel il est prévu un capteur à inertie (55), en particulier un inclinomètre, porté par l'élément de support allongé (40) et adapté pour détecter la valeur instantanée d'un angle alpha autour du premier axe horizontal d'inclinaison (50) et la valeur instantanée d'un angle bêta autour du second axe horizontal panoramique (52) par rapport à une référence; le signal produit par le capteur à inertie (55) est envoyé à ladite unité de commande à microprocesseur (57).

3. Système selon la revendication 1 ou 2, dans lequel l'unité de commande à microprocesseur (57) est portée par l'élément de support (40).

4. Système selon l'une quelconque des revendications précédentes, dans lequel l'élément de support allongé (40) comprend une paroi allongée (60) comprenant une partie centrale rectangulaire plane (60-c) et des parties d'extrémité plates (60-a, 60-b) disposées inclinées par rapport à la partie centrale (60-c); chaque partie d'extrémité (60-a, 60-b) est pourvue d'un trou traversant (62-a, 62-b) dans lequel est logé un ensemble respectif comprenant la première / seconde caméra vidéo (42-a, 42-b) et le premier / deuxième illuminant (43-a, 43-b); la première extrémité du dispositif de pivotement motorisé (45) est reliée de manière stable à la partie centrale (60-c).

5. Système selon la revendication 4, dans lequel chaque ensemble comprend une paroi de support plate (64) sous la forme d'une couronne circulaire supportant, sur une première face de celle-ci, les DELs (65) de l'illuminant et munie d'une caméra vidéo montée coaxialement à la paroi de support de structure plate en forme de couronne circulaire (64).

6. Système selon la revendication 4 ou 5, dans lequel le dispositif de pivotement motorisé (45) comprend un premier support en forme de L (70) muni d'une première partie plate avec un côté plus court (70-a) fixé sur la partie centrale (60-b) et une seconde partie plate avec un côté plus long (70-b) qui porte un premier actionneur (73) de type électrique pourvu d'un premier élément de sortie rotatif (74) qui tourne autour du premier axe horizontal d'inclinaison (50); une partie d'extrémité du premier élément de sortie en rotation (74) est fixée à une première partie plate d'un second support (75) et qui comprend une seconde partie plate perpendiculaire à la première et à une partie d'extrémité d'un second élément de sortie rotatif d'un second actionneur électrique (77); le second élément de sortie rotatif s'étend depuis un boîtier du second actionneur (77) et tourne autour du second axe horizontal panoramique (52); le boîtier du second actionneur (77) est solidaire d'un élément qui constitue la seconde extrémité du système de pivotement motorisé (45).

7. Système selon l'une quelconque des revendications précédentes, dans lequel ledit capteur optique de localisation (20) est pourvu d'un premier générateur adapté pour émettre un rayon laser LS selon une première direction qui est transversale à l'élément de support allongé (40) et coupe la zone de visualisation (FOV); le rayon laser LS a une longueur d'onde visible à l'oeil humain et est prévu pour produire un point laser visible sur le corps du patient P qui peut être utilisé dans une procédure de réglage du capteur optique de localisation (20).

8. Système selon la revendication 7, dans lequel ledit capteur optique de localisation (20) est pourvu d'un second générateur adapté pour émettre un rayon infrarouge le long d'une seconde direction qui est transversale à l'élément de support allongé (40) et coupe la zone de visualisation (FOV) affectant le patient (P); le système comprend également un dispositif de déviation qui produit le mouvement du rayon infrarouge par rapport à la structure de support (7) de manière à produire un point infrarouge qui frappe une zone du patient et se déplace dans l'espace en effectuant le balayage de cette zone; les caméras vidéo (42-a, 42-b) sont adaptées pour détecter la position dans l'espace (x, y, z) de ce point et l'unité de commande détecte la distance entre le capteur optique de position (20) et la position dans l'espace (x, y, z) du point; l'unité de commande (57) est adaptée pour corréler les valeurs de distance mesurées pour les positions adjacentes successives du point et répéter les balayages pour des sections contiguës les unes aux autres afin d'obtenir un profil tridimensionnel de la zone d'intérêt.

9. Système selon la revendication 8, dans lequel le second générateur de rayons infrarouges appartient à un système optique solidaire de l'élément de support allongé (40) et émet un faisceau infrarouge ayant une inclinaison prédéterminée par rapport à l'élément de support allongé (40); le dispositif de déviation est issu du dispositif de pivotement motorisé (45) qui effectue la rotation de l'élément de support allongé (40) autour du premier axe horizontal d'inclinaison (50) et du second axe horizontal panoramique (52) pour régler la position dans l'espace du faisceau infrarouge et déplacer le point infrarouge à la zone du patient afin d'effectuer un balayage.

10. Système selon la revendication 9, dans lequel le système optique comprend:
- une source (100-a) configurée pour émettre un faisceau infrarouge qui est alimenté à une première entrée d'un diviseur de faisceau (105-a);
- une source laser (110-b) qui émet un faisceau de lumière visible qui est alimenté à une seconde entrée du diviseur de faisceau (105-a) après avoir traversé un générateur de motif (115-a); le diviseur de faisceau (105-a) est configuré pour délivrer en sortie le faisceau de rayons infrarouges et le faisceau laser ayant des directions de propagation parallèles l'une à l'autre.

11. Système selon la revendication 8, dans lequel le générateur de rayons infrarouges est solidaire de l'élément de support allongé (40) et émet un faisceau infrarouge ayant une inclinaison réglable par rapport à l'élément de support allongé (40) au moyen de la déviation produite par un système optique porté par l'élément de support allongé (40) et configuré pour ajuster la position dans l'espace du faisceau infrarouge pour déplacer le point infrarouge sur la zone du patient en effectuant un balayage.

12. Système selon la revendication 11, dans lequel le second générateur comprend:
- une source (100-b) configurée pour émettre un faisceau de rayons infrarouges qui est alimenté à une première entrée d'un diviseur de faisceau (105-b);
- une source laser (110-b) qui émet un faisceau de lumière visible qui est alimenté à une seconde entrée du diviseur de faisceau (105-b); le diviseur de faisceau (105-b) est configuré pour délivrer le faisceau de rayons infrarouges et le faisceau laser ayant des directions de propagation parallèles l'une à l'autre;
- un miroir oscillant (117-b) sur lequel le faisceau infrarouge et le faisceau laser provenant de la sortie du diviseur de faisceau sont incidents; le miroir oscillant est configuré pour pivoter autour de l'axe horizontal d'inclinaison (50) et autour de l'axe horizontal panoramique (52).

13. Système selon la revendication 12, dans lequel un générateur de motif (115-b) est interposé entre la source laser (110-b) et la seconde entrée du diviseur de faisceau (105-b).
